# EUROPEAN PATENT SPECIFICATION

(11) **EP 3 400 869 B1**
(45) Date of publication and mention of the grant of the patent: **30.10.2019**
(21) Application number: 18171932.9
(22) Date of filing: 11.05.2018
(51) Int. Cl.: A61B 5/05, G01R 33/12, G01R 33/38, G01R 33/383, A61B 5/00

(54) **MAGNETIC PARTICLE IMAGING (MPI) DEVICE**
MAGNETISCHE PARTIKELABBILDUNGSVORRICHTUNG
DISPOSITIF D'IMAGERIE À PARTICULES MAGNÉTIQUES

(30) Priority: 12.05.2017 IT 201700051917
(43) Date of publication of application: 14.11.2018
(73) Proprietor: MASMEC S.p.A., 70026 Modugno (IT)
(72) Inventor: LARIZZA, Pietro, 70026 MODUGNO (BA) (IT)
(74) Representative: Bongiovanni, Simone

(56) References cited:
- US-A1- 2012 310 076
- US-A1- 2015 008 910
- BUZUG T M ET AL: "Novel hardware developments in magnetic particle imaging", MEDICAL IMAGING 2011: BIOMEDICAL APPLICATIONS IN MOLECULAR, STRUCTURAL, AND FUNCTIONAL IMAGING, SPIE, 1000 20TH ST. BELLINGHAM WA 98225-6705 USA, vol. 7965, no. 1, 3 March 2011 (2011-03-03), pages 1-6, XP060008562, DOI: 10.1117/12.877158

## Description

The present invention relates to an MPI (Magnetic Particle Imaging) device.

Magnetic particle imaging technology was first presented in 2005 by authors Gleich and Weizenecker (B. Gleich, and R. Weizenecker) in "Tomographic imaging using the nonlinear response of magnetic particles", Nature, 435 (2005), pp. 1214-1217).

Some prototypes of imaging devices have now been developed of the type comprising a first magnetic control system configured to generate a low-frequency magnetic field in a three-dimensional measuring space and obtaining, within said three-dimensional space, the movement along three axes X, Y and Z of a spot in which the field has a value lower than a threshold value. The MPI imaging device further comprises a second magnetic excitation system adapted to generate a pulsating high-frequency magnetic field within this spot so as to excite iron oxide microparticles injected into the circulatory system of a patient having a portion of his or her body positioned in said three-dimensional measuring space. MPI analysis also employs an antenna system adapted to detect the response of the iron oxide microparticles.

By analysing the signal detected by the antenna it is actually possible to map a three-dimensional image of portions of the human body permeated by blood vessels, as the portion of the body where blood vessels are present corresponds to the portion of the body where the iron oxide microparticles oscillate due to the high-frequency magnetic excitation. Iron oxide microparticles outside the spot cannot oscillate at high frequency as they are polarized by the strong magnetic field produced by the magnetic control system.

Until now, the proposed experimental imaging devices provide a measuring space that is entirely surrounded by magnetic deflection systems that make access to the patient impossible when he or she is positioned, totally or partially, within the measuring space.

Document US20157008910 discloses a Magnetic Particle Imaging (MPI) device.

The present invention is intended to resolve the aforementioned technical problem.

This technical problem is overcome by the present invention in so far as it relates to a Magnetic Particle Imaging device comprising a first magnetic control system configured to generate a magnetic field inside a three-dimensional measuring space and to obtain, within this three-dimensional space, the movement in three directions X, Y, and Z of a spot wherein the field has a value lower than a threshold, said Magnetic Particle Imaging device further comprising a second magnetic excitation system configured to generate a pulsating magnetic field within said spot so as to excite iron oxide microparticles injected into the circulatory system of a patient having a portion of his or her body positioned in said three-dimensional measuring space, said magnetic control system comprises: a first bottom parallelepipedal permanent magnet; a second top parallelepipedal permanent magnet spaced apart from the first magnet in the vertical direction Z, the first permanent magnet and the second permanent magnet having mutually facing poles, coaxial magnetic axes, and being arranged on opposite sides of the three-dimensional measuring space; a first pair of first and second electromagnets arranged respectively facing a first and a second side face of the first bottom permanent magnet, the first and the second side faces being arranged on opposite sides of the first parallelepipedal permanent magnet, said first pair of electromagnets being configured to generate, when powered, a magnetic field that combines with the magnetic field generated by the permanent magnets and modifies the spatial arrangement of the lines of force of the field along a first horizontal direction X; and a second pair of third and fourth electromagnets arranged respectively facing a third and a fourth face of the first, bottom, permanent magnet, the third and the fourth faces being arranged on opposite sides of the first, parallelepipedal, permanent magnet, said second pair of electromagnets being configured to generate, when powered, a magnetic field that combines with the magnetic field generated by the permanent magnets and modifies the spatial arrangement of the lines of force of the field in a second horizontal direction Y; said first, parallelepipedal, permanent magnet having a fifth, top, face facing the three-dimensional measuring space and a sixth, bottom, face facing a base portion of the device; said magnetic control system further comprising a fifth electromagnet with its axis coaxial with the axes of the first and the second permanent magnets, said fifth electromagnet being adapted to generate, when powered, a magnetic field that combines with the magnetic field generated by the permanent magnets and modifies the spatial arrangement of the lines of force of the field along the vertical direction Z.

The invention will now be described with reference to the accompanying drawings, which show a preferred non-limitative embodiment, in which:
Figure 1 shows an exploded, perspective view of a device for MPI imaging embodied according to the teachings of the present invention;
Figure 2 shows a side view of a device for MPI imaging embodied according to the teachings of the present invention;
Figure 3 shows a perspective view of a device for MPI imaging embodied according to the teachings of the present invention;
Figures 4 and 5 show control devices of the device according to the present invention; and
Figure 6 shows a schematic circuit diagram of the magnetic part of the device according to the present invention.

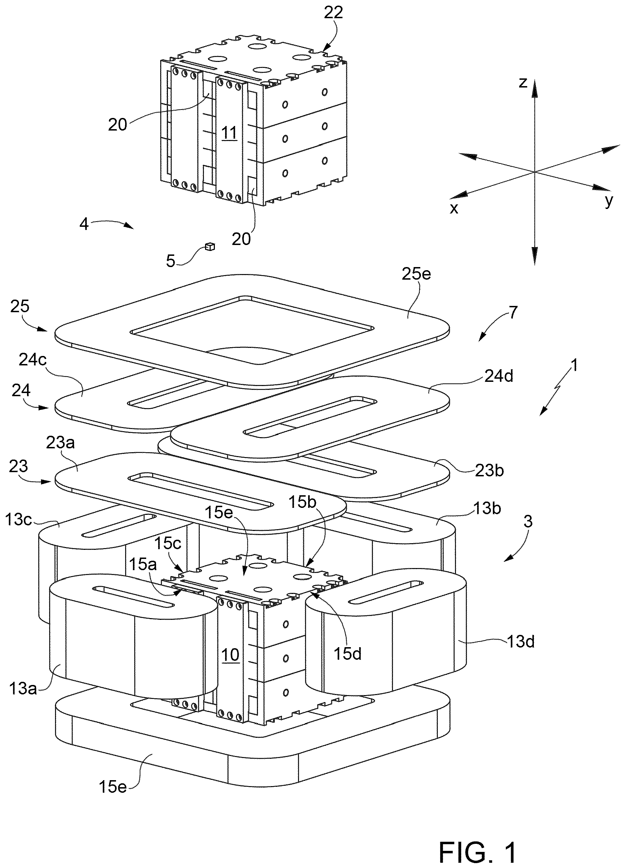

In Figure 1, a device for MPI imaging (Magnetic Particle Imaging) is indicated by reference numeral 1.

The device 1 comprises a first magnetic control system 3 configured to generate a magnetic field in a three-dimensional measuring space 4 and obtain, within this three-dimensional space 4, the movement in three directions X, Y and Z of a spot 5 within which the field has a value considerably lower than a threshold. Typically, the spot 5 can have an approximately spherical shape and a diameter of 1 mm or lower. The three-dimensional space 4 can be approximately cubic with sides 0.8-1 metre long.

The device 1 further comprises a second magnetic excitation system 7 adapted to generate a pulsating magnetic field within the spot 5 so as to excite iron oxide microparticles injected into the circulatory system of a patient (not shown for simplicity) having a portion of his or her body positioned in the three-dimensional measuring space 4.

According to the present invention, the first magnetic control system 3 comprises a first bottom parallelepipedal permanent magnet 10 (its shape is cubic in the example), and a second top parallelepipedal permanent magnet 11 (its shape is cubic in the example) vertically spaced apart from the first magnet 10 in respect to the vertical direction Z. The spacing between the two magnets 10 and 11 can be in the order of approximately 1 metre. The first permanent magnet 10 and the second permanent magnet 11 have mutually facing poles of the same polarity, coaxial magnetic axes, and are arranged on opposite sides of the three-dimensional measuring space 4.

The first magnetic control system 1 further comprises a first pair of first and second electromagnets 13a and 13b with axes parallel to the axes of the permanent magnets 10 and 11 in the direction Z, arranged respectively facing a first and a second side face 15a and 15b of the first bottom permanent magnet 10; the first and the second side faces 15a and 15b are arranged on opposite sides of the first parallelepipedal permanent magnet 10. The first pair of electromagnets 13a and 13b is adapted to generate, when powered, a magnetic field that combines with the magnetic field generated by the permanent magnets 10 and 11 and modifies the spatial arrangement of the lines of force of the field along a first horizontal direction X.

The first magnetic control system 1 comprises a second pair of third and fourth electromagnets 13c and 13d with axes parallel to the axes of the permanent magnets 10 and 11 in the direction Z, arranged respectively facing a third and a fourth side face 15c and 15d of the first bottom permanent magnet 10; the third and the fourth side faces 15c and 15d are arranged on opposite sides of the first parallelepipedal permanent magnet 10. The second pair of electromagnets 13c and 13d is adapted to generate, when powered, a magnetic field that combines with the magnetic field generated by the permanent magnets 10 and 11 and modifies the spatial arrangement of the lines of force of the field along a second horizontal direction Y.

The first parallelepipedal permanent magnet 10 has a fifth top face 15e facing the three-dimensional measuring space 4 and a sixth bottom face (not visible in Figure 1) facing a base portion of the device 1.

Finally, the magnetic control system 1 comprises a fifth electromagnet 25e with its axis coaxial with the axes of the first and the second permanent magnets 10 and 11; the fifth electromagnet 25e is adapted to generate, when powered, a magnetic field that combines with the magnetic field generated by the permanent magnets 10 and 11 and modifies the spatial arrangement of the lines of force of the field along the vertical direction Z.

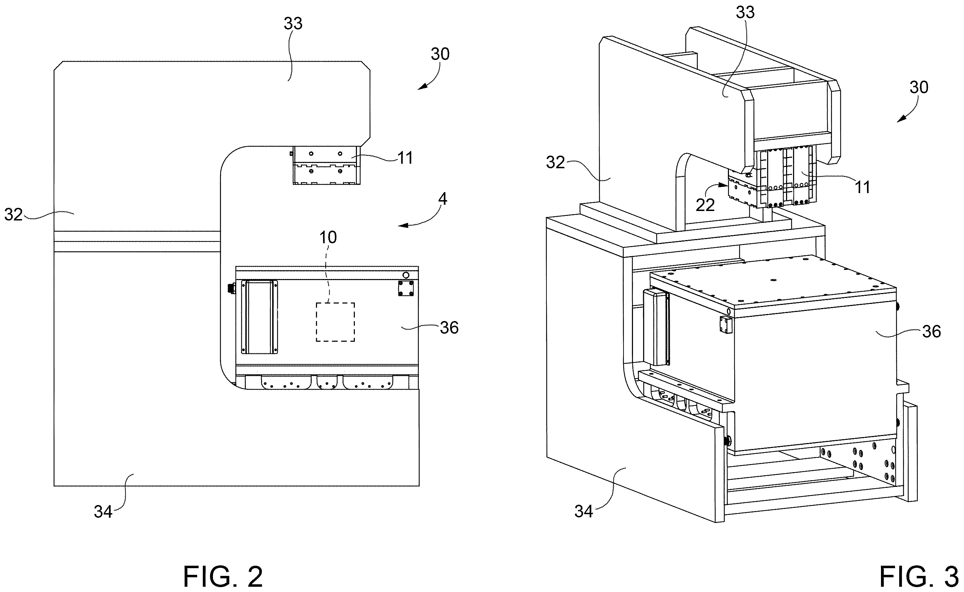

The first and the second permanent magnets 10 and 11 comprise a plurality of magnetic modules 20 arranged alongside one another so as to produce a cubic structure housed in a casing 22 (Figure 3) made of non-magnetic material configured to withstand the repulsive forces generated by the magnetic modules 20 arranged with the corresponding poles facing one another. Magnetic modules 20 made of neodymium-iron-boron (NdFeB) can be expediently used, the which, as is known, represent one of the best magnetic materials currently used in the industry.

The casing 22 can be expediently made of reinforced fibreglass, Kevlar, carbon fibre or a high-resistance plastic material. It is opportune to avoid the use of any metal material (for example, metal screws).

The first, second, third and fourth electromagnets 13a, 13b, 13c and 13d are devoid of a metal core and each comprise a coil with an insulated conductor wound about a hollow central portion inside which, in use, a cooling liquid circulates (this aspect will be described further on).

The insulated conductor is expediently formed by a strand of enameled copper wires (litz wire) which is used in order to reduce the "skin effect", reducing the resistance of the AC current circulating in the coil.

The magnetic excitation system 7 comprises a plurality of electromagnets, the coils of which develop in planes perpendicular to the direction Z between the fifth face 15e of the first permanent magnet 10 and a bottom side of the three-dimensional measuring space 4.

In greater detail, the magnetic excitation system 7 comprises a layered structure comprising:
a first bottom layer 23, in which there is a first and a second excitation electromagnet 23a and 23b arranged alongside one another and having axes parallel to the direction Z; the field formed by the first and the second electromagnets 23a and 23b is adapted to create lines of force that move along the first direction X;
a second intermediate layer 24, in which there is a third and a fourth excitation electromagnet 24c and 24d arranged alongside one another and having axes parallel to the direction Z; the field formed by the third and the fourth electromagnets 24c and 24d being adapted to create lines of force that move along the second direction Y; and
a third top layer 25, in which there is a fifth excitation electromagnet 25e having its axis parallel to the direction Z; the field formed by the fifth electromagnet 25e is adapted to create lines of force that move in the third direction Z.

The first and the second electromagnets 23a and 23b are shaped in plan view like a rectangular frame with rounded corners and arranged with their longer sides parallel to one another, the third and the fourth electromagnets 24c and 24d are shaped in plan view like a rectangular frame with rounded corners and arranged with their longer sides parallel to one another and perpendicular to the longer sides of the first and the second electromagnets 23a and 23b, and the fifth electromagnet 25e is rectangular in plan view, in particular square, and is arranged with a first pair of sides parallel to the longer sides of the first and the second electromagnets 23a and 23b and with a second pair of sides parallel to the longer sides of the third and the fourth electromagnets 24c and 24d.

The third layer 25 is set close to and facing the three-dimensional measuring space 4, the first layer 23 is set close to and facing the fifth face 15e of the bottom permanent magnet, and the second layer 24 is interposed between the first layer 23 and the third layer 25.

The particular embodiment described above enables creating a measuring space 4 that is laterally open 360 degrees (the measuring space 4 is only delimited by the magnet 11 above and delimited below by the magnet 10 and by the coils belonging to the second magnetic excitation system 7 on top of the bottom magnet 10). Medical personnel can thus work unhindered on a patient with a portion of his or her body positioned in the measuring space.

This arrangement constitutes an evident advantage with respect to known devices, in which the measuring space is totally surrounded by magnetic deflection systems that make access to the patient impossible when he or she is positioned, totally or partially, in the measuring space.

Figures 2 and 3 show a supporting and containing structure 30 for supporting the first magnetic control system 3 and the magnetic excitation system 7 that is made entirely of non-magnetic material (for example, in a high-resistance plastic material, generically known as "engineering plastics"). The supporting structure 30 is formed by a substantially C-shaped portion comprising a vertical upright 32, a top transverse arm 33 adapted to support the second, top, permanent magnet 11, and a bottom structure 34 adapted to support the first bottom permanent magnet 10 and all the electromagnets that belong to the first magnetic control system 3 and to the magnetic excitation system 7.

The bottom structure 34 supports a fluid-tight container 36 made of non-magnetic material that defines an inner chamber (not shown), which houses the first bottom permanent magnet 10 and all the electromagnets that belong to the first magnetic control system 3 and to the magnetic excitation system 7. A cooling system (of known type and not shown) is also provided, this being adapted to enable a flow of insulating cooling fluid (for example, transformer oil) between a supply inlet of the fluid-tight container 36 and a discharge outlet of the fluid-tight container 36. In this way, the cooling fluid can flow around the electromagnets and circulate through the portions of the electromagnets devoid of a metal core.

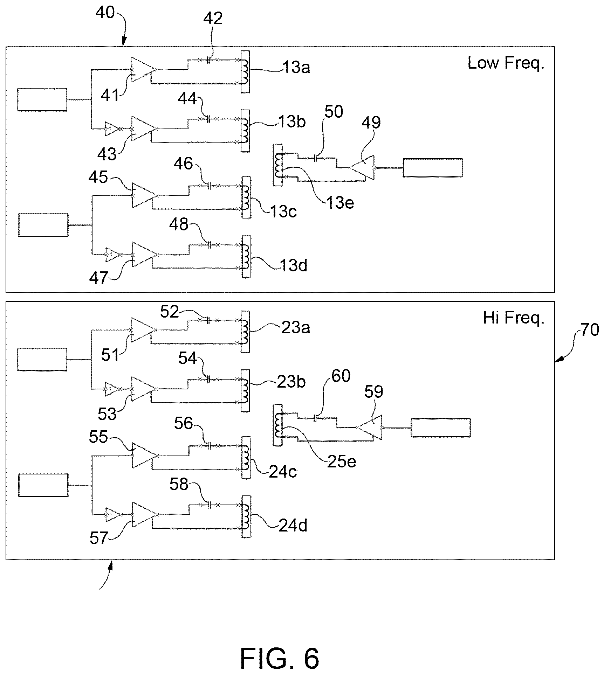

Figure 6 shows a simplified wiring diagram of the low-frequency power-supply system 40 for powering the first magnetic control system 3, comprising:
a first power generator 41 (with a working frequency ranging between 0.1 Hz and 500 Hz) that supplies an AC signal to a first LC network comprising the first electromagnet 13a, which constitutes the inductive component, and a first battery of capacitors 42, which constitutes the capacitive component;
a second power generator 43 (with a working frequency ranging between 0.1 Hz and 500 Hz) that supplies an AC signal to a second LC network comprising the second electromagnet 13b, which constitutes the inductive component, and a second battery of capacitors 44, which constitutes the capacitive component;
a third power generator 45 (with a working frequency ranging between 0.1 Hz and 500 Hz) that supplies an AC signal to a third LC network comprising the third electromagnet 13c, which constitutes the inductive component, and a third battery of capacitors 46, which constitutes the capacitive component;
a fourth power generator 47 (with a working frequency ranging between 0.1 Hz and 500 Hz) that supplies an AC signal to a fourth LC network comprising the fourth electromagnet 13d, which constitutes the inductive component, and a fourth battery of capacitors 48, which constitutes the capacitive component; and
a fifth power generator 49 (with a working frequency ranging between 0.1 Hz and 500 Hz) that supplies an AC signal to a fifth LC network comprising the fifth electromagnet 13e, which constitutes the inductive component, and a fifth battery of capacitors 50, which constitutes the capacitive component.

The first and the second power generators 41 and 43 receive as input the same excitation signal, but with opposite phases, and the third and the fourth power generators 45 and 47 receive as input the same excitation signal, but with opposite phases.

The capacitors 42, 44, 46, 48 and 50 are expediently carried by the bottom structure 34 and are arranged beneath the fluid-tight container 36.

Similarly, a high-frequency power supply system 70 is provided for the magnetic excitation system 7 comprising:
a first power generator 51 (with a working frequency ranging between 8 KHz and 30 KHz) that supplies an AC signal to a first LC network comprising the first electromagnet 23a, which constitutes the inductive component, and a first battery of capacitors 52, which constitutes the capacitive component;
a second power generator 53 (with a working frequency ranging between 8 KHz and 30 KHz) that supplies an AC signal to a second LC network comprising the second electromagnet 23b, which constitutes the inductive component, and a second battery of capacitors 54, which constitutes the capacitive component;
a third power generator 55 (with a working frequency ranging between 8 KHz and 30 KHz) that supplies an AC signal to a third LC network comprising the third electromagnet 24c, which constitutes the inductive component, and a third battery of capacitors 56, which constitutes the capacitive component;
a fourth power generator 57 (with a working frequency ranging between 8 KHz and 30 KHz) that supplies an AC signal to a fourth LC network comprising the fourth electromagnet 24d, which constitutes the inductive component, and a fourth battery of capacitors 58, which constitutes the capacitive component; and
a fifth power generator 59 (with a working frequency ranging between 8 KHz and 30 KHz) that supplies an AC signal to a fifth LC network comprising the fifth electromagnet 25e, which constitutes the inductive component, and a fifth battery of capacitors 60, which constitutes the capacitive component.

The first and the second power generators 51 and 53 receive as input the same excitation signal, but with opposite phases, and the third and the fourth power generators 55 and 57 receive as input the same excitation signal, but with opposite phases.

The capacitors 52, 55, 56, 58 and 59 are expediently carried by the bottom structure 34 and are arranged beneath the fluid-tight container 36.

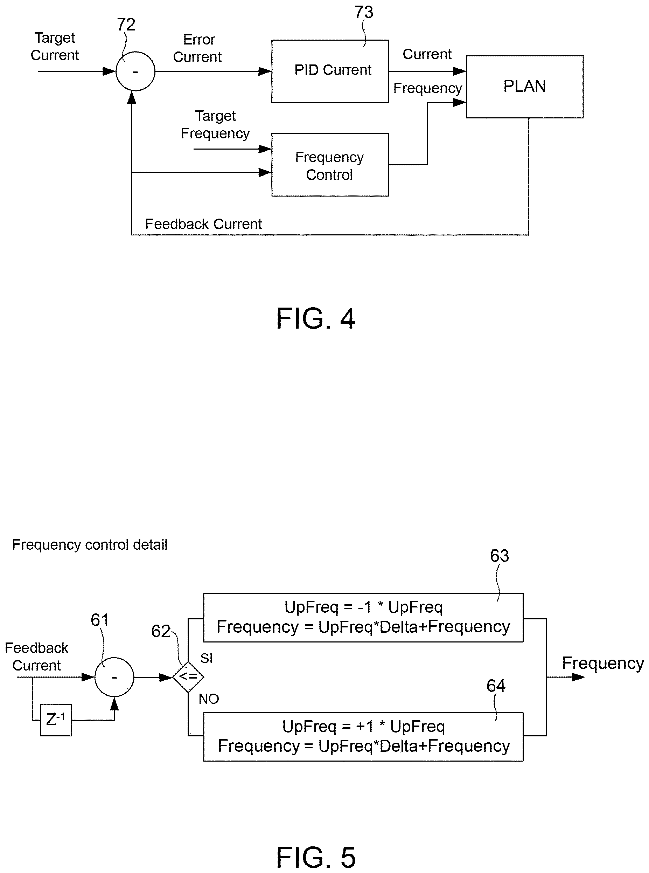

Each LC network is provided with a frequency-control system adapted to supply it, by means of the respective power generator 41, 43, 45, 47, 49 and 51, 53, 55, 57, 59, with an AC signal having a frequency close to the resonant frequency of the LC network:
The frequency-control system is schematically shown in Figure 5 and comprises:
an adder block 61 adapted to produce a present sample of the value of the feedback current at a certain frequency of the AC signal supplied to the LC network and a sample of the value of feedback current sampled at a previous sampling instant and hence at a previous working frequency;
a comparator 62 adapted to examine the value output from the adder block by comparing it with the zero value;
a first corrector block 63 adapted to vary, in a discrete manner, the value of the frequency of the signal generated by the power generator in the case where the comparator 62 arrives at a first determination of less than or equal to zero; and
a second corrector block 64 adapted to vary, in a discrete manner, the value of the frequency of the signal generated by the power generator in the case where the comparator arrives at a second determination greater than zero.

Figure 4 shows a system for controlling the current within each LC network, this current-control system implementing a control loop external to the control loop provided by the frequency-control system that drives the frequency of a sinusoidal waveform generator that determines the frequency of the sinusoid that powers each LC network.

The current-control system comprises:
a second comparator 72 adapted to check the mean value of current supplied to each LC network against a target value; and
a PID regulator 73, which receives as input the value of the error signal output from the second comparator and supplies a driving signal for a sine-wave generator that determines the amplitude of the sinusoid that powers each LC network.

The invention is defined by appended claims 1-15.

## Claims

1. A Magnetic Particle Imaging, MPI, device comprising a first magnetic control system (3) configured to generate, in a three-dimensional measuring space (4), a magnetic field and obtain within said three-dimensional space (4) the movement in three directions X, Y, and Z of a spot (5), wherein the field has a value lower than a threshold, said Magnetic Particle Imaging, MPI, device further comprising a second magnetic excitation system (7) configured to generate a pulsating magnetic field within said spot (5) and configured to excite iron oxide microparticles injected into the circulatory system of a patient having a portion of his or her body positioned in said three-dimensional measuring space, said magnetic control system comprises:
a first bottom parallelepipedal permanent magnet (10),
a second top parallelepipedal permanent magnet (11) spaced apart from the first magnet (10) in the vertical direction Z; the first permanent magnet (10) and the second permanent magnet (11) having mutually facing poles of the same polarity, sharing the same magnetic axes, and being arranged on opposite sides of the three-dimensional measuring space (4);
a first pair of first and second electromagnets (13a, 13b) arranged respectively facing a first side face (15a) and a second side face (15b) of the first bottom permanent magnet (10), the first and the second side faces (15a, 15b) being arranged on opposite sides of the first parallelepipedal permanent magnet (10); said first pair of electromagnets (13a, 13b) being configured to generate, when powered, a magnetic field that combines with the magnetic field generated by the permanent magnets (10, 11) and modifies the spatial arrangement of the lines of force of the field along a first horizontal direction X; and
a second pair of third and fourth electromagnets (13c, 13d) arranged respectively facing a third face (15c) and a fourth face (15d) of the first bottom permanent magnet (10), the third and the fourth faces (15c, 15d) being arranged on opposite sides of the first parallelepipedal permanent magnet (10); said second pair of electromagnets (13c, 13d) being adapted to generate, when powered, a magnetic field that combines with the magnetic field generated by the permanent magnets (10, 11) and modifies the spatial arrangement of the lines of force of the field in a second horizontal direction Y;
said first, parallelepipedal, permanent magnet (10) having a fifth, top, face (15e) facing the three-dimensional measuring space (4) and a sixth, bottom, face facing a base portion of the device;
said magnetic control system (3) further comprising a fifth electromagnet (25e) with axes coaxial to the axes of the first and the second permanent magnets (10, 11), said fifth electromagnet (25e) being configured to generate, when powered, a magnetic field that combines with the magnetic field generated by the permanent magnets (10, 11) and modifies the spatial arrangement of the lines of force of the field along the vertical direction Z.

2. The device according to Claim 1, wherein the magnetic excitation system (7) comprises a plurality of electromagnets, the coils of which extend in planes perpendicular to the direction Z between the fifth top face (15e) of the first permanent magnet (10) and a bottom side of the three-dimensional measuring space (4) .

3. The device according to Claim 2, wherein the magnetic excitation system (7) comprises a layered structure comprising:
a first layer (23), in which there is a first excitation electromagnet (23a) and a second excitation electromagnet (23b) arranged alongside one another and having axes parallel to the direction Z; the field generated by the first and by the second electromagnets being adapted to create lines of force that move in the first direction X;
a second layer (24), in which there is a third excitation electromagnet (24c) and a fourth excitation electromagnet (24d) arranged alongside one another and having axes parallel to the direction Z; the field generated by the third and the fourth electromagnets (24c, 24d) being configured to create lines of force that move along the second direction Y;
a third layer (25), in which there is a fifth excitation electromagnet (25e) having its axis parallel to direction Z; the field generated by the fifth electromagnet (25e) being adapted to create lines of force that move in the third direction Z.

4. The device according to Claim 3, wherein the first and the second electromagnets (23a, 23b) are shaped in plan view as a rectangular frame with rounded corners arranged with their longer sides parallel to one another, the third and the fourth electromagnets (24c, 24d) are shaped in plan view like a rectangular frame with rounded corners arranged with their longer sides parallel to one another and perpendicular to the longer sides of the first and the second electromagnets (23a, 23b), and the fifth electromagnet (25e) is rectangular in plan view, in particular square, and is arranged with a first pair of sides parallel to the longer sides of the first and the second electromagnets (23a, 23b) and with a second pair of sides parallel to the longer sides of the third and the fourth electromagnets (24c, 24d).

5. The device according to Claim 3 or Claim 4, wherein the third layer (25) is set close to and facing the three-dimensional measuring space (4), the first layer (23) is set close to and facing the fifth face (15e) of the bottom permanent magnet, and the second layer (24) is interposed between the first layer and the third layer.

6. The device according to any one of the preceding claims, wherein the first and the second permanent magnets (10, 11) have a cubic shape.

7. The device according to any one of the preceding claims, wherein the first and the second permanent magnets (10, 11) comprise a plurality of separate magnetic modules (20) arranged alongside one another in such a way as to obtain a cubic structure housed in a casing (22) made of non-magnetic material configured to withstand the repulsive forces generated by the magnetic modules arranged with corresponding poles facing one another.

8. The device according to any one of the preceding claims, wherein the first, second, third, and fourth electromagnets (13a, 13b, 13c, and 13d) are without a metal core and each comprise a coil of insulated conductive wire wound around a hollow central portion.

9. The device according to any one of the preceding claims, wherein the first, second, third, fourth, and fifth electromagnets (13a, 13b, 13c, 13d, and 13e) each comprise a coil of insulated conductive wire formed by a strand of wires.

10. The device according to any one of the preceding claims, wherein a supporting structure (30) for supporting the first magnetic control system (3) and the magnetic excitation system (7) is provided, which is made entirely of non-magnetic material; said supporting structure (30) comprising a substantially C-shaped portion comprising a vertical upright (32), a top transverse arm (33) adapted to support the second top permanent magnet (11), and a bottom structure (34) adapted to support the first bottom permanent magnet (10) and all the electromagnets that belong to the first magnetic control system (3) and to the magnetic excitation system (7).

11. The device according to Claim 10, wherein the bottom structure supports a fluid-tight container (36) made of non-magnetic material, which houses the first bottom permanent magnet (10) and all the electromagnets that belong to the first magnetic control system (3) and to the magnetic excitation system (7): being moreover provided with a cooling system configured to enable a flow of insulating cooling fluid between a supply inlet of the fluid-tight container (36) and a discharge outlet of the fluid-tight container (36).

12. The device according to any one of the preceding claims, wherein a low-frequency power-supply system (40) for powering the first magnetic control system (3) is provided, comprising:
a first power generator (41) that supplies an AC signal to a first LC network comprising the first electromagnet (13a), which constitutes the inductive component, and a first battery of capacitors (42), which constitutes the capacitive component;
a second power generator (43) that supplies an AC signal to a second LC network comprising the second electromagnet (13b), which constitutes the inductive component, and a second battery of capacitors (44), which constitutes the capacitive component;
a third power generator (45) that supplies an AC signal to a third LC network comprising the third electromagnet (13c), which constitutes the inductive component, and a third battery of capacitors (46), which constitutes the capacitive component;
a fourth power generator (47) that supplies an AC signal to a fourth LC network comprising the fourth electromagnet (13d), which constitutes the inductive component, and a fourth battery of capacitors (48), which constitutes the capacitive component; and
a fifth power generator (49) that supplies an AC signal to a fifth LC network including the fifth electromagnet (13e), which constitutes the inductive component, and a fifth battery of capacitors (50), which constitutes the capacitive component.

13. The device according to Claim 12, wherein the first and the second power generators (41, 43) receive as input the same excitation signal with opposite phases, and the third and the fourth power generators (45, 47) receive as input the same excitation signal with opposite phases.

14. The device according to Claim 12 or Claim 13, wherein each LC network is provided with a frequency-control system adapted to supply to the network, by means of the respective power generator (41, 43, 45, 47, 49 and 51, 53, 55, 57, 59), with an AC signal having a frequency close to the resonant frequency of the LC network:
said frequency-control system comprises:
an adder block (61) adapted to produce a present sample of the value of a feedback current at a certain frequency of the AC signal supplied to the LC network and a sample of the value of feedback current sampled at a previous sampling instant and hence at a previous working frequency;
a comparator (62) configured to examine the value output from the adder block by comparing it with the zero value;
a first corrector block (63) configured to vary, in a discrete manner, the value of the frequency of the signal generated by the power generator in the case where the comparator (62) arrives at a first determination of less than or equal to zero;
a second corrector block (64) adapted to vary, in a discrete manner, the value of the frequency of the signal generated by the power generator in the case where the comparator arrives at a second determination greater than zero.

15. The device according to Claim 14, wherein a system for controlling the current within each LC network is provided; said current-control system constituting a control loop external to the control loop provided by the frequency-control system that drives the frequency of a sinusoidal waveform generator that determines the frequency of the sinusoid that powers each LC network;
the current-control system comprising:
a second comparator (72) configured to check the mean value of current supplied to each LC network against a threshold value; and a PID regulator (73), which receives as input the value of the error signal output from the second comparator and supplies a driving signal for the respective power generator.

## Patentansprüche

1. Vorrichtung zur Magnetpartikelbildgebung, Magnetic Particle Imaging, MPI, mit einem ersten Magnetsteuersystem (3), das so konfiguriert ist, dass es in einem dreidimensionalen Messraum (4) ein Magnetfeld erzeugt und im dreidimensionalen Raum (4) die Bewegung eines Flecks (5) in drei Richtungen X, Y und Z erhält, wobei das Feld einen niedrigeren Wert als ein Schwellwert hat, wobei die Vorrichtung zur Magnetpartikelbildgebung, MPI, ferner ein zweites Magneterregungssystem (7) aufweist, das so konfiguriert ist, dass es ein pulsierendes Magnetfeld im Fleck (5) erzeugt, und so konfiguriert ist, dass es Eisenoxid-Mikropartikel anregt, die in das Kreislaufsystem eines Patienten injiziert sind, von dem ein Abschnitt seines Körpers im dreidimensionalen Messraum positioniert ist, wobei das Magnetsteuersystem aufweist:
einen ersten unteren parallelepipeden Dauermagnet (10),
einen zweiten oberen parallelepipeden Dauermagnet (11), der vom ersten Magnet (10) in Vertikalrichtung Z beabstandet ist; wobei der erste Dauermagnet (10) und der zweite Dauermagnet (11) zueinander weisende Pole mit gleicher Polarität haben, die gleichen Polachsen gemeinsam haben und auf Gegenseiten des dreidimensionalen Messraums (4) angeordnet sind;
ein erstes Paar aus einem ersten und einem zweiten Elektromagnet (13a, 13b), die zu einer ersten Seitenfläche (15a) bzw. einer zweiten Seitenfläche (15b) des ersten unteren Dauermagneten (10) weisend angeordnet sind, wobei die erste und zweite Seitenfläche (15a, 15b) auf Gegenseiten des ersten parallelepipeden Dauermagneten (10) angeordnet sind; wobei das erste Paar Elektromagnete (13a, 13b) so konfiguriert ist, dass es bei Energetisierung ein Magnetfeld erzeugt, das sich mit dem durch die Dauermagnete (10, 11) erzeugten Magnetfeld kombiniert und die räumliche Anordnung der Kraftlinien des Felds entlang einer ersten Horizontalrichtung X modifiziert; und
ein zweites Paar aus einem dritten und einem vierten Elektromagnet (13c, 13d), die zu einer dritten Fläche (15c) bzw. einer vierten Fläche (15d) des ersten unteren Dauermagneten (10) weisend angeordnet sind, wobei die dritte und vierte Fläche (15c, 15d) auf Gegenseiten des ersten parallelepipeden Dauermagneten (10) angeordnet sind; wobei das zweite Paar Elektromagnete (13c, 13d) geeignet ist, bei Energetisierung ein Magnetfeld zu erzeugen, das sich mit dem durch die Dauermagnete (10, 11) erzeugten Magnetfeld kombiniert und die räumliche Anordnung der Kraftlinien des Felds in einer zweiten Horizontalrichtung Y modifiziert;
wobei der erste, parallelepipede Dauermagnet (10) eine fünfte Kopffläche (15e), die zum dreidimensionalen Messraum (4) weist, und eine sechste Bodenfläche hat, die zu einem Sockelabschnitt der Vorrichtung weist;
wobei das Magnetsteuersystem (3) ferner einen fünften Elektromagnet (25e) mit Achsen koaxial zu den Achsen des ersten und zweiten Dauermagneten (10, 11) aufweist und der fünfte Elektromagnet (25e) so konfiguriert ist, dass er bei Energetisierung ein Magnetfeld erzeugt, das sich mit dem durch die Dauermagnete (10, 11) erzeugten Magnetfeld kombiniert und die räumliche Anordnung der Kraftlinien des Felds entlang der Vertikalrichtung Z modifiziert.

2. Vorrichtung nach Anspruch 1, wobei das Magneterregungssystem (7) mehrere Elektromagnete aufweist, deren Spulen sich in Ebenen senkrecht zur Richtung Z zwischen der fünften Kopffläche (15e) des ersten Dauermagneten (10) und einer Unterseite des dreidimensionalen Messraums (4) erstrecken.

3. Vorrichtung nach Anspruch 2, wobei das Magneterregungssystem (7) über einen Schichtaufbau verfügt, der aufweist:
eine erste Schicht (23), in der ein erster Erregerelektromagnet (23a) und ein zweiter Erregerelektromagnet (23b) liegen, die nebeneinander angeordnet sind und Achsen parallel zur Richtung Z haben; wobei das durch den ersten und durch den zweiten Elektromagnet erzeugte Feld geeignet ist, Kraftlinien zu erzeugen, die sich in der ersten Richtung X bewegen;
eine zweite Schicht (24), in der ein dritter Erregerelektromagnet (24c) und ein vierter Erregerelektromagnet (24d) liegen, die nebeneinander angeordnet sind und Achsen parallel zur Richtung Z haben; wobei das durch den dritten und vierten Elektromagnet (24c, 24d) erzeugte Feld so konfiguriert ist, dass es Kraftlinien erzeugt, die sich entlang der zweiten Richtung Y bewegen;
eine dritte Schicht (25), in der ein fünfter Erregerelektromagnet (25e) liegt, dessen Achse parallel zur Richtung Z ist; wobei das durch den fünften Elektromagnet (25e) erzeugte Feld geeignet ist, Kraftlinien zu erzeugen, die sich in der dritten Richtung Z bewegen.

4. Vorrichtung nach Anspruch 3, wobei der erste und zweite Elektromagnet (23a, 23b) in Draufsicht als Rechteckrahmen mit abgerundeten Ecken geformt sind, die mit ihren längeren Seiten parallel zueinander angeordnet sind, der dritte und vierte Elektromagnet (24c, 24d) in Draufsicht wie ein Rechteckrahmen mit abgerundeten Ecken geformt sind, die mit ihren längeren Seiten parallel zueinander und senkrecht zu den längeren Seiten des ersten und zweiten Elektromagneten (23a, 23b) angeordnet sind, und der fünfte Elektromagnet (25e) in Draufsicht rechteckig, insbesondere quadratisch ist und mit einem ersten Paar Seiten parallel zu den längeren Seiten des ersten und zweiten Elektromagneten (23a, 23b) und mit einem zweiten Paar Seiten parallel zu den längeren Seiten des dritten und vierten Elektromagneten (24c, 24d) angeordnet ist.

5. Vorrichtung nach Anspruch 3 oder Anspruch 4, wobei die dritte Schicht (25) nahe am dreidimensionalen Messraum (4) und zu ihm weisend platziert ist, die erste Schicht (23) nahe an der fünften Fläche (15e) des unteren Dauermagneten und zu ihr weisend platziert ist und die zweite Schicht (24) zwischen der ersten Schicht und der dritten Schicht eingefügt ist.

6. Vorrichtung nach einem der vorstehenden Ansprüche, wobei der erste und zweite Dauermagnet (10, 11) eine Würfelform haben.

7. Vorrichtung nach einem der vorstehenden Ansprüche, wobei der erste und zweite Dauermagnet (10, 11) mehrere separate Magnetmodule (20) aufweisen, die nebeneinander so angeordnet sind, dass ein Würfelaufbau zustande kommt, der in einem Gehäuse (22) aufgenommen ist, das aus einem nichtmagnetischen Material hergestellt ist, das so konfiguriert ist, dass es den Abstoßungskräften wiedersteht, die durch die mit entsprechenden Polen zueinander weisend angeordneten Magnetmodule erzeugt werden.

8. Vorrichtung nach einem der vorstehenden Ansprüche, wobei der erste, zweite, dritte und vierte Elektromagnet (13a, 13b, 13c und 13d) keinen Metallkern haben und jeweils eine Spule aus isoliertem leitenden Draht aufweisen, der um einen hohlen Mittelabschnitt gewickelt ist.

9. Vorrichtung nach einem der vorstehenden Ansprüche, wobei der erste, zweite, dritte, vierte und fünfte Elektromagnet (13a, 13b, 13c, 13d und 13e) jeweils eine Spule aus isoliertem leitenden Draht aufweisen, der durch eine Drahtlitze gebildet ist.

10. Vorrichtung nach einem der vorstehenden Ansprüche, wobei ein Stützaufbau (30) zum Stützen des ersten Magnetsteuersystems (3) und des Magneterregungssystems (7) vorgesehen ist, der vollständig aus nichtmagnetischem Material hergestellt ist; wobei der Stützaufbau (30) aufweist: einen im Wesentlichen C-förmigen Abschnitt mit einem vertikalen Ständer (32), einem oberen Querarm (33), der geeignet ist, den zweiten oberen Dauermagnet (11) zu stützen, und einem unteren Aufbau (34), der geeignet ist, den ersten unteren Dauermagnet (10) und alle Elektromagnete zu stützen, die zum ersten Magnetsteuersystem (3) und zum Magneterregungssystem (7) gehören.

11. Vorrichtung nach Anspruch 10, wobei der untere Aufbau einen aus nichtmagnetischem Material hergestellten fluiddichten Behälter (36) stützt, der den ersten unteren Dauermagnet (10) und alle Elektromagnete aufnimmt, die zum ersten Magnetsteuersystem (3) und zum Magneterregungssystem (7) gehören; wobei er außerdem mit einem Kühlsystem versehen ist, das so konfiguriert ist, dass es einen Durchfluss von nicht leitendem Kühlfluid zwischen einem Zufuhreinlass des fluiddichten Behälters (36) und einem Abgabeauslass des fluiddichten Behälters (36) ermöglicht.

12. Vorrichtung nach einem der vorstehenden Ansprüche, wobei ein niederfrequentes Stromversorgungssystem (40) zum Energetisieren des ersten Magnetsteuersystems (3) vorgesehen ist, das aufweist:
einen ersten Leistungsgenerator (41), der ein Wechselstromsignal einer ersten LC-Schaltung zuführt, die den ersten Elektromagnet (13a), der die induktive Komponente bildet, und eine erste Batterie aus Kondensatoren (42) aufweist, die die kapazitive Komponente bildet;
einen zweiten Leistungsgenerator (43), der ein Wechselstromsignal einer zweiten LC-Schaltung zuführt, die den zweiten Elektromagnet (13b), der die induktive Komponente bildet, und eine zweite Batterie aus Kondensatoren (44) aufweist, die die kapazitive Komponente bildet;
einen dritten Leistungsgenerator (45), der ein Wechselstromsignal einer dritten LC-Schaltung zuführt, die den dritten Elektromagnet (13c), der die induktive Komponente bildet, und eine dritte Batterie aus Kondensatoren (46) aufweist, die die kapazitive Komponente bildet;
einen vierten Leistungsgenerator (47), der ein Wechselstromsignal einer vierten LC-Schaltung zuführt, die den vierten Elektromagnet (13d), der die induktive Komponente bildet, und eine vierte Batterie aus Kondensatoren (48) aufweist, die die kapazitive Komponente bildet; und
einen fünften Leistungsgenerator (49), der ein Wechselstromsignal einer fünften LC-Schaltung zuführt, die den fünften Elektromagnet (13e), der die induktive Komponente bildet, und eine fünfte Batterie aus Kondensatoren (50) aufweist, die die kapazitive Komponente bildet.

13. Vorrichtung nach Anspruch 12, wobei der erste und zweite Leistungsgenerator (41, 43) als Eingabe das gleiche Anregungssignal mit entgegengesetzten Phasen empfangen und der dritte und vierte Leistungsgenerator (45, 47) als Eingabe das gleiche Anregungssignal mit entgegengesetzten Phasen empfangen.

14. Vorrichtung nach Anspruch 12 oder Anspruch 13, wobei jede LC-Schaltung mit einem Frequenzsteuersystem versehen ist, das geeignet ist, der Schaltung mit Hilfe des jeweiligen Leistungsgenerators (41, 43, 45, 47, 49 und 51, 53, 55, 57, 59) ein Wechselstromsignal mit einer Frequenz nahe der Resonanzfrequenz der LC-Schaltung zuzuführen;
wobei das Frequenzsteuersystem aufweist:
einen Addierblock (61), der geeignet ist, eine aktuelle Abtastung des Werts eines Rückkopplungsstroms mit einer bestimmten Frequenz des der LC-Schaltung zugeführten Wechselstromsignals und eine Abtastung des Werts des Rückkopplungsstroms zu erzeugen, der zu einer vorherigen Abtastinstanz und somit mit einer vorherigen Arbeitsfrequenz abgetastet wurde;
einen Komparator (62), der so konfiguriert ist, dass er den vom Addierblock ausgegebenen Wert durch dessen Vergleichen mit dem Nullwert prüft;
einen ersten Korrektorblock (63), der so konfiguriert ist, dass er auf diskrete Weise den Wert der Frequenz des durch den Leistungsgenerator erzeugten Signals in dem Fall variiert, in dem der Komparator (62) bei einer ersten Bestimmung von weniger oder gleich null ankommt;
einen zweiten Korrektorblock (64), der geeignet ist, auf diskrete Weise den Wert der Frequenz des durch den Leistungsgenerator erzeugten Signals in dem Fall zu variieren, in dem der Komparator bei einer zweiten Bestimmung von größer null ankommt.

15. Vorrichtung nach Anspruch 14, wobei ein System zum Steuern des Stroms in jeder LC-Schaltung vorgesehen ist, wobei das Stromsteuersystem einen Steuerkreis außerhalb des Steuerkreises bildet, der durch das Frequenzsteuersystem vorgesehen ist, das die Frequenz eines Sinuswellenformgenerators ansteuert, der die Frequenz der Sinuslinie bestimmt, die jede LC-Schaltung speist;
wobei das Stromsteuersystem aufweist:
einen zweiten Komparator (72), der so konfiguriert ist, dass er den Mittelwert von Strom, der jeder LC-Schaltung zugeführt wird, mit einem Schwellwert vergleicht; und
einen PID-Regler (73), der als Eingabe den Wert des vom zweiten Komparator ausgegebenen Fehlersignals empfängt und ein Ansteuersignal den jeweiligen Leistungsgenerator zuführt.

## Revendications

1. Dispositif MPI d'imagerie à particules magnétiques comprenant un premier système de commande magnétique (3) configuré pour générer, dans un espace de mesure tridimensionnel (4), un champ magnétique et obtenir à l'intérieur dudit espace tridimensionnel (4) le mouvement dans trois directions X , Y et Z d'un spot (5), dans lequel le champ a une valeur inférieure à un seuil, ledit dispositif MPI d'imagerie à particules magnétiques comprenant en outre un second système d'excitation magnétique (7) configuré pour générer un champ magnétique pulsé à l'intérieur dudit spot (5) et configuré pour exciter des microparticules d'oxyde de fer injectées dans le système circulatoire d'un patient ayant une partie de son corps positionnée dans ledit espace de mesure tridimensionnel, ledit système de commande magnétique comprenant :
un premier aimant permanent inférieur parallélépipédique (10),
un deuxième aimant permanent supérieur parallélépipédique (11) espacé du premier aimant (10) dans la direction verticale Z; le premier aimant permanent (10) et le deuxième aimant permanent (11) ayant des pôles de la même polarité se faisant face, partageant les mêmes axes magnétiques, et étant disposés sur les côtés opposés de l'espace de mesure tridimensionnel (4);
une première paire de premier et second électroaimants (13a, 13b) agencés respectivement en regard d'une première face latérale (15a) et d'une seconde face latérale (15b) du premier aimant permanent inférieur (10), les première et seconde faces latérales (15a , 15b) étant disposés sur les côtés opposés du premier aimant permanent parallélépipédique (10); ladite première paire d'électroaimants (13a, 13b) étant adaptée pour générer, lorsqu'elle est alimentée, un champ magnétique qui se combine avec le champ magnétique généré par les aimants permanents (10, 11) et modifie la disposition spatiale des lignes de force du champ selon une première direction horizontale X; et une seconde paire de troisième et quatrième électroaimants (13c, 13d) disposés respectivement en regard d'une troisième face (15c) et d'une quatrième face (15d) du premier aimant permanent inférieur (10), les troisième et quatrième faces (15c, 15d) étant disposées sur les côtés opposés du premier aimant permanent parallélépipédique (10); ladite seconde paire d'électroaimants (13c, 13d) étant adaptée pour générer, lorsqu'elle est alimentée, un champ magnétique qui se combine avec le champ magnétique généré par les aimants permanents (10, 11) et modifie la disposition spatiale des lignes de force du champ dans une seconde direction horizontale Y;
ledit premier aimant permanent parallélépipédique (10) ayant une cinquième face supérieure (15e) faisant face à l'espace de mesure tridimensionnel (4) et une sixième face inférieure en regard d'une partie de base du dispositif;
ledit système de commande magnétique (3) comprenant en outre un cinquième électroaimant (25e) ayant des axes coaxiaux aux axes des premier et deuxième aimants permanents (10, 11), ledit cinquième électroaimant (25e) étant adapté pour générer, lorsqu'il est alimenté, un champ magnétique qui se combine avec le champ magnétique généré par les aimants permanents (10, 11) et modifie la disposition spatiale des lignes de force du champ dans la direction verticale Z.

2. Dispositif selon la revendication 1, dans lequel le système d'excitation magnétique (7) comprend une pluralité d'électroaimants dont les bobines s'étendent dans des plans perpendiculaires à la direction Z entre la cinquième face supérieure (15e) du premier aimant permanent (10) et un côté inférieur de l'espace de mesure tridimensionnel (4).

3. Dispositif selon la revendication 2, dans lequel le système d'excitation magnétique (7) comprend une structure en couches comprenant:
une première couche (23), dans laquelle se trouvent un premier électroaimant d'excitation (23a) et un second électroaimant d'excitation (23b) agencés le long l'un de l'autre et ayant des axes parallèles à la direction Z; le champ généré par le premier et le deuxième électroaimants étant adapté pour créer des lignes de force qui se déplacent dans la première direction X;
une deuxième couche (24) dans laquelle se trouvent un troisième électroaimant d'excitation (24c) et un quatrième électroaimant d'excitation (24d) disposés côte à côte et ayant des axes parallèles à la direction Z; le champ généré par les troisième et quatrième électroaimants (24c, 24d) étant adapté pour créer des lignes de force qui se déplacent dans la seconde direction Y;
une troisième couche (25) dans laquelle se trouve un cinquième électroaimant d'excitation (25e) d'axe parallèle à la direction Z; le champ généré par le cinquième électroaimant (25e) étant adapté pour créer des lignes de force qui se déplacent dans la troisième direction Z.

4. Dispositif selon la revendication 3, dans lequel les premier et deuxième électroaimants (23a, 23b) ont une forme en vue de dessus comme un cadre rectangulaire avec des coins arrondis agencés avec leurs côtés les plus longs parallèles l'un à l'autre, les troisième et quatrième électroaimants (24c, 24d)) ont une forme en vue de dessus comme un cadre rectangulaire avec des coins arrondis disposés avec leurs côtés les plus longs parallèles les uns aux autres et perpendiculaires aux côtés les plus longs des premier et deuxième électroaimants (23a, 23b), et le cinquième électroaimant (25e) est rectangulaire en vue de dessus, en particulier carrée, et est agencé avec une première paire de côtés parallèles aux côtés les plus longs des premier et deuxième électroaimants (23a, 23b) et avec une seconde paire de côtés parallèles aux côtés les plus longs du troisième et du quatrièmes électroaimants (24c, 24d).

5. Dispositif selon la revendication 3 ou 4, dans lequel la troisième couche (25) est placée à proximité de et en regard de l'espace de mesure tridimensionnel (4), la première couche (23) est fixée à proximité de la cinquième face (15e) de l'aimant permanent inférieur, et la deuxième couche (24) est interposée entre la première couche et la troisième couche.

6. Dispositif selon l'une quelconque des revendications précédentes, dans lequel les premier et deuxième aimants permanents (10, 11) ont une forme cubique.

7. Dispositif selon l'une quelconque des revendications précédentes, dans lequel les premier et deuxième aimants permanents (10, 11) comprennent une pluralité de modules magnétiques séparés (20) agencés les uns à côté des autres de manière à obtenir une structure cubique logée dans un boîtier (22) en matériau non magnétique configuré pour résister aux forces de répulsion générées par les modules magnétiques agencés avec des pôles correspondants se faisant face.

8. Dispositif selon l'une quelconque des revendications précédentes, dans lequel les premier, deuxième, troisième et quatrième électroaimants (13a, 13b, 13c et 13d) sont sans âme métallique et comprennent chacun une bobine de fil conducteur isolé enroulé autour d'une partie centrale creuse.

9. Dispositif selon l'une quelconque des revendications précédentes, dans lequel les premier, deuxième, troisième, quatrième et cinquième électroaimants (13a, 13b, 13c, 13d et 13e) comprennent chacun une bobine de fil conducteur isolé formé par un brin de fils.

10. Dispositif selon l'une quelconque des revendications précédentes, dans lequel une structure de support (30) pour supporter le premier système de commande magnétique (3) et le système d'excitation magnétique (7) est prévue, qui est entièrement constituée d'un matériau non magnétique; ladite structure de support (30) comprenant une partie sensiblement en forme de C comprenant un montant vertical (32), un bras transversal supérieur (33) adapté pour supporter le second aimant permanent supérieur (11), et une structure inférieure (34) adaptée pour supporter le premier aimant permanent inférieur (10) et tous les électroaimants appartenant au premier système de commande magnétique (3) et au système d'excitation magnétique (7).

11. Dispositif selon la revendication 10, dans lequel la structure inférieure supporte un conteneur (36) étanche aux fluides en matériau non magnétique, qui loge le premier aimant permanent inférieur (10) et tous les électroaimants appartenant au premier système de commande magnétique (3) et au système d'excitation magnétique (7): comportant en outre un système de refroidissement adapté pour permettre un écoulement de fluide de refroidissement isolant entre une entrée d'alimentation du conteneur étanche aux fluides (36) et une sortie de décharge du fluide étanche aux fluides conteneur (36).

12. Dispositif selon l'une quelconque des revendications précédentes, dans lequel un système d'alimentation en énergie basse fréquence (40) pour alimenter le premier système de commande magnétique (3) est prévu, comprenant:
un premier générateur d'énergie (41) qui fournit un signal alternatif à un premier réseau LC comprenant le premier électroaimant (13a), qui constitue le composant inductif, et une première batterie de condensateurs (42), qui constitue le composant capacitif;
un second générateur de puissance (43) qui fournit un signal alternatif à un second réseau LC comprenant le second électroaimant (13b), qui constitue le composant inductif, et une seconde batterie de condensateurs (44), qui constitue le composant capacitif;
un troisième générateur de puissance (45) qui fournit un signal alternatif à un troisième réseau LC comprenant le troisième électroaimant (13c), qui constitue le composant inductif, et une troisième batterie de condensateurs (46), qui constitue le composant capacitif;
un quatrième générateur de puissance (47) qui fournit un signal alternatif à un quatrième réseau LC comprenant le quatrième électroaimant (13d), qui constitue le composant inductif, et une quatrième batterie de condensateurs (48), qui constitue le composant capacitif; et un cinquième générateur de puissance (49) qui fournit un signal alternatif à un cinquième réseau LC comprenant le cinquième électroaimant (13e), qui constitue le composant inductif, et une cinquième batterie de condensateurs (50), qui constitue le composant capacitif.

13. Dispositif selon la revendication 12, dans lequel les premier et second générateurs de puissance (41, 43) reçoivent en entrée le même signal d'excitation avec des phases opposées, et les troisième et quatrième générateurs de puissance (45, 47) reçoivent en entrée le même signal d'excitation avec des phases opposées.

14. Dispositif selon la revendication 12 ou la revendication 13, dans lequel chaque réseau LC est doté d'un système de commande de fréquence adapté pour l'alimenter au moyen du générateur de puissance respectif (41, 43, 45, 47, 49 et 51, 53, 55, 57, 59), avec un signal alternatif ayant une fréquence proche de la fréquence de résonance du réseau LC:
ledit système de commande de fréquence comprenant :
un bloc additionneur (61) adapté pour produire un échantillon actuel de la valeur d'un courant de retour à une certaine fréquence du signal alternatif fourni au réseau LC et un échantillon de la valeur du courant de retour échantillonné à un instant d'échantillonnage précédent et donc à une fréquence de travail précédente;
un comparateur (62) adapté pour examiner la valeur délivrée par le bloc additionneur en le comparant à la valeur zéro;
un premier bloc correcteur (63) adapté pour faire varier de manière discrète la valeur de la fréquence du signal généré par le générateur de puissance dans le cas où le comparateur (62) arrive à une première détermination inférieure ou égale à zéro ;
un deuxième bloc correcteur (64) adapté pour faire varier de manière discrète la valeur de la fréquence du signal généré par le générateur de puissance dans le cas où le comparateur arrive à une deuxième détermination supérieure à zéro.

15. Dispositif selon la revendication 14, dans lequel un système pour contrôler le courant dans chaque réseau LC est prévu; ledit système de commande de courant constituant une boucle de commande externe à la boucle de commande fournie par le système de commande de fréquence qui commande la fréquence d'un générateur de forme d'onde sinusoïdale qui détermine la fréquence de la sinusoïde qui alimente chaque réseau LC;
le système de commande de courant comprenant:
un deuxième comparateur (72) configuré pour vérifier la valeur moyenne du courant fourni à chaque réseau LC par rapport à une valeur de seuil; et un régulateur PID (73), qui reçoit en entrée la valeur du signal d'erreur délivré par le deuxième comparateur et fournit un signal de commande pour le générateur de puissance respectif.
